# EUROPÄISCHE PATENTSCHRIFT

(11) **EP 1 415 159 B1**
(45) Veröffentlichungstag und Bekanntmachung des Hinweises auf die Patenterteilung: **24.10.2007**
(21) Anmeldenummer: 02751167.4
(22) Anmeldetag: 02.08.2002
(51) Int. Cl.: G01N 33/62, G01N 33/58, C12Q 1/04

(54) **VERFAHREN UND DIAGNOSEKIT ZUR DIAGNOSE VON HELICOBACTER PYLORI**
METHOD AND DIAGNOSTIC KIT FOR DIAGNOSING HELICOBACTER PYLORI
PROCEDE ET TROUSSE DE DIAGNOSTIC POUR DIAGNOSTIQUER UN HELICOBACTER PYLORI

(30) Priorität: 09.08.2001 DE 10139299
(43) Veröffentlichungstag der Anmeldung: 06.05.2004
(73) Patentinhaber: Aygen, Sitke, 51105 Köln (DE)
(72) Erfinder: Aygen, Sitke, 51105 Köln (DE)
(74) Vertreter: Schreiber, Christoph
(86) Internationale Anmeldenummer: PCT/EP2002/008611
(87) Internationale Veröffentlichungsnummer: WO 2003/014744

(56) Entgegenhaltungen:
- EP-A- 0 881 491
- DE-U- 29 613 373
- US-A- 5 542 419
- US-A- 5 928 167
- US-A- 6 113 875
- W.D. CHEY ET AL: "The 13C-urea blood test accurately detects active Helicobacter pylori infection: a US, Multicenter trial" AMERICAN JOURNAL OF GASTROENTEROLOGY, Bd. 94, Nr. 6, 1999, Seiten 1522-1524, XP002245806 in der Anmeldung erwähnt
- A.F. CUTLER & P. TOSKES: "comparison of 13c-urea blood test to 13c-urea breath test for the diagnosis of helicobacter pylori" THE AMERICAN JOURNAL OF GASTROENTEROLOGY, Bd. 94, Nr. 4, 1999, Seiten 959-961, XP002245807 in der Anmeldung erwähnt

## Beschreibung

Gegenstand der vorliegenden Erfindung ist ein Verfahren zur Diagnose von Helicobacter pylori durch orale Applikation von definierten Mengen von C₁₃ markiertem Harnstoff und Untersuchung von zu einem definierten Zeitpunkt entnommenen Blutproben auf den C₁₃-Gehalt.

Das bisher übliche und am meisten durchgeführte Verfahren zur Diagnose von Helicobacter pylori ist der C₁₃-Harnstoff Atemtest. Dieses Verfahren ist ausführlich beschrieben in der EP-A-0 253 927. Dieser Test hat aber den Nachteil, dass er nicht bei Kindern unter 3 Jahren und bei Patienten mit Atembeschwerden wie Asthma durchgeführt werden kann. **Ein Atemtest ist auch in** EP 0 881 491 A1 **beschrieben.**

Rex Moulton-Barrett et al., The American Journal of Gastroenterology, Vol. 88, 1993, Seiten 369 bis 374 beschreiben ein Verfahren, bei welchem nach oraler Applikation von C₁₃ markiertem Harnstoff C₁₃ markiertes Bicarbonat im Serum bestimmt wird. **Ein ähnliches Verfahren ist in** US 5,542,419 **offenbart.** Bei diesem Test wurden dem Patienten 5 mg/kg C₁₃ markierter Harnstoff verabreicht und je 3 ml Blutproben nach 15, 30, 60, 90, 120 und 180 min abgenommen und untersucht. Diese Methode ist weiter untersucht und beschrieben worden von Mark J. Kim et al. in Gastroenterology 1997; 113:31 - 37, W.D. Chey et al. in The American Journal of Gastroenterology, Vol. 94, 1999, Seiten 1522 bis 1524, Alan F. Cuttler et al. in The American Journal of Gastroenterology, Vol. 94, 1999, Seiten 959 bis 961 und hat zur Einführung eines Testes geführt, von der Firma Metabolic Solutions Inc., Nashua NH. Bei diesem Test wird ohne Bestimmung des Nullwertes nach Applikation einer fettreichen Probemalzeit "Ensure" 125 mg C₁₃-Harnstoff appliziert und nach 30 min aus 3 ml Blut der C₁₃-Gehalt bestimmt. Dieser Test hat aber wegen der nur geringen Genauigkeit und Präzision einerseits, des sehr hohen Preises für die notwendige Menge C₁₃-Harnstoff sowie der relativ großen Testmenge an Blut wirtschaftlich zu keinem Erfolg geführt, so dass der Atemtest trotz all seiner Nachteile die meistbenutzte Testmethode blieb.

Die Erfindung hat sich die Aufgabe gestellt, mit möglichst geringer Menge an C₁₃-Harnstoff und möglichst geringer Blutmenge zur Untersuchung des C₁₃-Gehalts ein Verfahren zur Diagnose von Helicobacter pylori zur Verfügung zu stellen, welches einfacher, billiger, genauer und schneller die Diagnose einer Infektion mit Helicobacter pylori feststellt.

Diese Aufgabe wird jetzt dadurch gelöst, dass vor Testbeginn von dem nüchternen Patienten 0,1 bis 0,6 ml Kapillarblut aus dem Finger oder Ohrläppchen oder venöses Blut entnommen wird, dem Patienten die exakte Menge von 10 bis 50 mg C₁₃-Harnstoff in wässriger Lösung mit einem pH-Wert von 2 bis 4 appliziert wird, exakt 10 bis 15 min nach der Applikation erneut Kapillarblut oder venöses Blut entnommen wird und von den Blutproben mittels Isotopen-Massenspektrometrie (IRMS) der C₁₃-Gehalt bestimmt und aus dem Anstieg der C₁₃-Werte auf das Vorhandensein von Helicobacter pylori geschlossen wird.

Selbst bei Verwendung von 0,6 ml Kapillarblut und 50 mg C₁₃-Harnstoff ist das Verfahren dem bisherigen Verfahren deutlich überlegen, zumal durch Bestimmung des Ausgangswertes die Genauigkeit des Verfahrens enorm ansteigt und durch Entnahme einer zweiten Blutprobe schon nach 10 bis 15 min das Verfahren für den Patienten deutlich verkürzt wird. Insbesondere bei älteren Patienten ist die Gewinnung von Kapillarblut manchmal mit Schwierigkeiten verbunden. Das Verfahren kann in solchen Fällen auch mit der entsprechenden geringen Menge venösem Blut durchgeführt werden.

Von entscheidender Bedeutung ist bei dem erfindungsgemäßen Verfahren der Wegfall der fettreichen Testmahlzeit. Stattdessen wird der markierte Harnstoff in wässriger Lösung mit einem pH-Wert von 2 bis 4 appliziert. Dies kann beispielsweise dadurch erfolgen, dass entweder die wässrige Lösung des markierten Harnstoffs mittels einer nicht flüchtigen, pharmakologisch unbedenklichen organischen Säure auf dem pH-Wert von 2 bis 4 eingestellt wird, oder der frisch zubereiteten Lösung des markierten Harnstoffs ein Päckchen einer festen pharmakologisch unbedenklichen organischen Säure zugesetzt wird. Besonders bewährt hat sich hierbei Zitronensäure. Prinzipiell sind aber auch andere derartige organische Säuren wie Ascorbinsäure, in der Lage, den gewünschten pH-Wert einzustellen. Es ist auch möglich, diesen niedrigen pH-Wert durch Verwendung von Orangensaft, Grapefruitsaft oder saurem Apfelsaft zu erreichen.

Die Bestimmung des C₁₃-Gehalts in den Blutproben kann beispielsweise erfolgen durch Zusatz einer starken, nicht flüchtigen Säure wie Phosphorsäure, die in der Lage ist, aus der Blutprobe das Kohlendioxid gasförmig freizusetzen, so dass es mit einem IRMS-Gerät gemessen werden kann.

Es ist aber auch möglich, aus den Blutproben Serum zu gewinnen, aus der Serumprobe mittel geeigneter Filter hochmolekulare Bestandteile zu entfernen und in der dann verbliebenen Flüssigkeit durch vorgeschaltete Elementaranalyse und nachfolgende Isotopen-Massenspektrometrie den C₁₃-Gehalt zu bestimmen.

Durch vergleichende Untersuchungen wurde festgestellt, dass der Nachweis einer Helicobacter pylori Infektion beim Atemtest vorliegt, wenn der Wert an C₁₃ in der Atemluft etwa 4°/°° über dem Ausgangswert liegt. Das erfindungsgemäße Verfahren ist demgegenüber in der Lage, Helicobacter-Infektionen schon nachzuweisen bei einer Differenz des C₁₃-Gehaltes von 2,0°/°°. Es leuchtet ein, dass die wesentlich geringere Menge des teuren C₁₃-Harnstoffs einerseits und die Verkürzung des Zeitpunkts der zweiten Probeentnahme von 30 min auf 10 min sowohl für die Patienten wie für die Ärzte von erheblicher Bedeutung sind. Darüber hinaus ist die Entnahme von maximal 0,6 ml Kapillarblut oder venösem Blut wesentlich einfacher und angenehmer als die Entnahme von 3 ml venösem Blut bei dem Test der Metabolic Solutions Inc. Gegenüber diesem Test ist vor allem auch die wesentlich höhere Genauigkeit und Präzision von entscheidender Bedeutung, da bei jedem Patienten der Ausgangswert bestimmt wird und nicht die mit erheblichen Schwankungen verbundenen Durchschnittswerte verwendet werden, welche durch unterschiedliche Nahrungsaufnahme der Probanten beeinflusst werden.

Der erfindungsgemäße Diagnosekit zur Durchführung des Verfahrens besteht vorzugsweise aus einer sauren wässrigen Lösung mit dem pH-Wert 2 bis 4, welche exakt 10 bis 50 mg C₁₃-Harnstoff enthält, einer Patienteninformation, zwei Probengefäßen zur Aufnahme der Blutproben und einer Blutentnahmevorrichtung. Entscheidend ist, dass der Diagnosekit eine genau eingewogene Menge C₁₃-Harnstoff enthält. Diese Menge kann in verschiedenen Diagnosekits beispielsweise für Kinder in dem Bereich zwischen 10 und 50 mg C₁₃-Harnstoff gewählt werden. Auch der Zeitraum zwischen den zwei Blutentnahmen sollte für einen bestimmten Diagnosekit exakt eingehalten werden, kann aber für verschiedene Diagnosekits im Bereich zwischen 10 und 15 min festgelegt werden.

Eine weitere bevorzugte Ausführungsform enthält anstelle der fertigen sauren Lösung des C₁₃-Harnstoffs einen Behälter für Harnstoff mit exakt 10 bis 50 mg, C₁₃-Harnstoff und ein Päckchen einer festen, pharmakologisch unbedenklichen organischen Säure wie Zitronensäure. 2 g Zitronensäure gelöst in 200 ml Wasser oder 200 ml Orangensaft, Grapefruitsaft oder saurer Apfelsaft sind geeignet, im Normalfall 30 bis 50 mg C₁₃-Harnstoff zu lösen. Insbesondere bei Kindern kann die Menge an C₁₃-Harnstoff weiter gesenkt werden bis auf 10 mg.

Die Blutproben werden vorzugsweise aufgenommen in handelsüblichen Probegefäßen zur Aufnahme der Blutproben, beispielsweise Vakutainer^{®}. Diese können vorzugsweise auch schon die notwendige Menge konzentrierte Phosphorsäure enthalten, so dass aus der Blutprobe unmittelbar das CO₂ freigesetzt wird. Prinzipiell ist es aber auch möglich, dem Probengefäß zur Aufnahme der Blutprobe nachträglich eine starke, nicht flüchtige Säure zuzusetzen.

Die Bestimmung des C₁₃-Gehaltes erfolgt dann mittels IRMS direkt aus der Gasphase. Bereits eine Zunahme des C₁₃-Gehaltes um 2°/°° nach 10 min zeigt eine Infektion mit Helicobacter pylori an.

Alternativ kann auch aus der Blutprobe nach kurzer Absetzzeit Serum abgezogen werden. Durch Ultrafiltration können aus dieser Serumprobe alle Makromoleküle und insbesondere die Lipide entfernt werden. Die dann verbliebene Flüssigkeit wird dann zunächst einer Elementaranalyse durch Verbrennung unterzogen und das dabei freigesetzte CO₂ wiederum durch IRMS auf das Isotopenverhältnis C₁₃/C₁₂ untersucht. Bei dieser Testmethode ist bereits die Infektion mit Helicobacter pylori feststellbar bei einem Anstieg des C₁₃-Gehaltes von 1 bis 1,5°/°°.

Die erfindungsgemäß erzielten höheren Empfindlichkeiten und Genauigkeiten der Testmethode lassen sich im Nachhinein dadurch erklären, dass der C₁₃-Gehalt des markierten Harnstoffs weniger verdünnt wird mit anderen Kohlenstoffquellen. Dieser Verdünnungseffekt ist am stärksten beim Atemlufttest und am geringsten bei der Untersuchung von Serumproben aus denen makromolekulare Moleküle durch Ultrafiltration entfernt werden. Dennoch ist bereits das Verfahren, bei dem aus der Blutprobe durch eine starke nicht flüchtige Säure CO₂ freigesetzt und in der Gasphase bestimmt wird, hervorragend geeignet, die gestellten Aufgaben der vorliegenden Erfindung zu erfüllen.

Bei der praktischen Durchführung des erfindungsgemäßen Verfahrens werden die Blutproben vor und 10 bis 15 min nach der Applikation des C₁₃-Hamstoffs vom Arzt durchführt. Die Vakutainer^{®} können gesammelt und an einem Zentrallabor geschickt werden, in welchem die Bestimmung der C₁₃-Gehalte erfolgt. Aus diesen dem Arzt zurückgemeldeten Ergebnissen kann der Arzt die Diagnose stellen, ob eine Infektion mit Helicobacter pylori vorliegt oder nicht.

Für die IRMS Bestimmung kommen prinzipiell alle auf den Markt befindlichen hochempfindlichen Isotopen-Massenspektrometer in Frage, die jedoch aufgrund ihrer Preise sich nur in Zentrallaboratorien aufstellen lassen. Die Investition derartiger Geräte ist bereits für Kliniken nicht zu erwarten. Das Einsammeln und Analysieren von Proben in Zentrallaboratorien hingegen ist heutzutage von der Logistik und von den Preisen her unproblematisch genauso wie die Rückübermittlung der Analysendaten an den einschickenden Arzt oder die einschickende Klinik.

Das gleiche gilt für die Bestimmungsmethode im Serum, aus welchem hochmolekulare Komponenten durch Ultrafiltration entfernt wurden. Die vorgeschaltete Elementaranalyse durch Verbrennung ist in Zentrallaboratorien ohne weiteres durchführbar. Die Gewinnung von Serum, aus Kapillarblut oder venösem Blut sowie die Ultrafiltration kann zumindest in Kliniken problemlos durchgeführt werden. Für die eigentliche Bestimmung des C₁₃-Gehaltes genügen 20 µl Serum und geben bereits bei einem Anstieg von 1 bis 1,5°/°° C₁₃ aussagekräftige Ergebnisse.

## Patentansprüche

1. Verfahren zur Diagnose von Helicobacter pylori durch orale Applikation von definierten Mengen von C₁₃-markiertem Harnstoff und Untersuchung von zu einem definierten Zeitpunkt entnommenen Blutproben auf den C₁₃-Gehalt, **dadurch gekennzeichnet, dass** in Blutproben von Patienten, denen
a) vor Testbeginn von dem nüchternen Patienten 0,1 bis 0,6 ml Kapillarblut aus dem Finger oder dem Ohrläppchen oder venöses Blut entnommen wurde,
b) dem Patienten die exakte Menge von 10 bis 50 mg C₁₃-Harnstoff in wässriger Lösung mit einem pH-Wert von 2 bis 4 appliziert wurde,
c) exakt 10 bis 15 min nach der Applikation erneut Kapillarblut oder venöses Blut entnommen wurde,
mittels Isotopen-Massenspektrometrie (IRMS) der C₁₃-Gehalt bestimmt und aus dem Anstieg der C₁₃-Werte auf das Vorhandensein von Helicobacter pylori geschlossen wird.

2. Verfahren gemäß Anspruch 1, **dadurch gekennzeichnet, dass** die Blutproben mit einer starken, nicht flüchtigen Säure versetzt werden, welche CO₂ gasförmig freisetzt und die C₁₃-Gehalte in der Gasphase bestimmt werden.

3. Verfahren gemäß Anspruch 1, **dadurch gekennzeichnet, dass** aus dem Blutproben Serum gewonnen wird, durch Filter aus dem Serum hochmolekulare Bestandteile entfernt werden und in der verbliebenen Flüssigkeit durch vorgeschaltete Elementaranalyse und nachfolgende Massenspektrometrie die C₁₃-Gehalte bestimmt werden.

4. Diagnosekit zur Durchführung des Verfahrens gemäß einem der Ansprüche 1 bis 3, bestehend aus
1) entweder einer sauren wässrigen Lösung mit dem pH-Wert 2 bis 4 enthaltend exakt 10 bis 50 mg C₁₃-Harnstoff oder
1a) einem Hamstoffbehälter mit exakt 10 bis 50 mg C₁₃-Harnstoff und
1b) einem Päckchen einer festen, pharmakologisch unbedenklichen organischen Säure,
2) einer Patienteninformation,
3) zwei Probengefäßen zur Aufnahme der Blutproben, welche bereits entweder
3a) eine starke, nicht flüchtige Säure enthalten oder
3b) den Zusatz einer solchen Säure ermöglichen,
4) eine Blutentnahmevorrichtung.

5. Diagnosekit gemäß Anspruch 4, **dadurch gekennzeichnet, dass** die organische Säure Zitronensäure ist.

## Claims

1. A method for the diagnosis of *Helicobacter pylori* infection by the oral administration of defined amounts of ¹³C-labeled urea and examination for ¹³C content of blood samples removed at a defined time, **characterized in that** in blood samples of patients from which
a) from 0.1 to 0.6 ml of capillary blood was removed from the finger or ear lob or venous blood of the patient with an empty stomach before the beginning of the test;
b) an exact amount of from 10 to 50 mg of ¹³C-urea in aqueous solution with a pH value of 2 to 4 was administered to the patient;
c) capillary blood or venous blood was again removed exactly after 10 to 15 min from the administration;
d) the ¹³C content is determined by isotope ratio mass spectrometry (IRMS), and the presence of *Helicobacter pylori* is deduced from the increase of the ¹³C values.

2. The method according to claim 1, **characterized in that** a strong non-volatile acid which releases CO₂ in a gaseous form is added to said blood samples, and the ¹³C contents are determined in the gas phase.

3. The method according to claim 1, **characterized in that** serum is recovered from the blood samples, high molecular weight components are removed from the serum by filters, and the ¹³C content is determined in the remaining liquid by a preliminary elemental analysis followed by mass spectrometry.

4. A diagnostic kit for performing the method according to any of claims 1 to 3, consisting of
1) either an acidic aqueous solution having a pH value of from 2 to 4 and containing exactly from 10 to 50 mg of ¹³C-urea, or
1a) a urea container with exactly from 10 to 50 mg of ¹³C-urea; and
1b) a pack of a solid pharmacologically acceptable organic acid;
2) a patient instruction sheet;
3) two sample vessels for receiving the blood samples which either
3a) already contain a strong non-volatile acid; or
3b) enable the addition of such an acid;
4) a blood-sampling device.

5. The diagnostic kit according to claim 4, **characterized in that** said organic acid is citric acid.

## Revendications

1. Procédé pour le diagnostic d'*Helicobacter pylori* au moyen d'une administration orale de quantités définies d'urée marquée au C¹³ et dosage de la teneur en C¹³ d'échantillons de sang prélevés à un moment défini, **caractérisé en ce que**, dans des échantillons de sang de patients chez lesquels
a) 0,1 à 0,6 mL de sang capillaire ont été prélevés avant le début de l'analyse chez le patient à jeun sur le doigt ou le lobe de l'oreille, ou du sang veineux a été prélevé,
b) le patient a reçu la quantité exacte de 10 à 50 mg d'urée C¹³ en solution aqueuse ayant un pH de 2 à 4,
c) du sang capillaire ou du sang veineux a été de nouveau prélevé exactement 10 à 15 minutes après l'administration,
la teneur en C¹³ est déterminée au moyen d'une spectrométrie de masse des rapports isotopiques (SMRI) et la présence d'*Helicobacter pylori* est déduite d'après l'augmentation des teneurs en C¹³.

2. Procédé selon la revendication 1, **caractérisé en ce que** les échantillons de sang sont mélangés avec un acide fort non volatil, lequel libère du CO₂ gazeux, et les teneurs en C¹³ sont déterminées en phase gazeuse.

3. Procédé selon la revendication 1, **caractérisé en ce que** du sérum est obtenu à partir des échantillons de sang, les constituants à haute masse moléculaire sont retirés du sérum à l'aide d'un filtre et les teneurs en C¹³ dans le liquide restant sont déterminées au moyen d'une analyse élémentaire réalisée en amont et d'une spectrométrie de masse réalisée en aval.

4. Trousse de diagnostic pour réaliser le procédé selon l'une quelconque des revendications 1 à 3, composée
1) d'une solution aqueuse acide ayant un pH de 2 à 4 contenant exactement 10 à 50 mg d'urée C¹³ ou
1a) un récipient d'urée contenant exactement 10 à 50 mg d'urée C¹³ et
1b) un petit sachet d'acide organique solide sans danger sur le plan pharmacologique,
2) d'une fiche d'information pour les patients,
3) de deux récipients pour échantillons devant contenir les échantillons de sang, qui
3a) contiennent déjà un acide fort non volatil ou
3b) permettent l'addition d'un tel acide,
4) d'un dispositif de prélèvement sanguin.

5. Trousse de diagnostic selon la revendication 4, **caractérisée en ce que** l'acide organique est l'acide citrique.
